(19) Europäisches Patentamt
European Patent Office
Office européen des brevets

(11) **EP 3 057 663 B1**

(12) **EUROPEAN PATENT SPECIFICATION**

(45) Date of publication and mention
of the grant of the patent:
**04.12.2019 Bulletin 2019/49**

(21) Application number: **14777627.2**

(22) Date of filing: **01.10.2014**

(51) Int Cl.:
*A61Q 5/00* (2006.01)        *A61Q 5/02* (2006.01)
*A61Q 5/12* (2006.01)        *A61K 8/73* (2006.01)
*A61K 8/06* (2006.01)

(86) International application number:
**PCT/EP2014/071016**

(87) International publication number:
**WO 2015/055432 (23.04.2015 Gazette 2015/16)**

(54) **HAIR CARE COMPOSITION**

HAARPFLEGEZUSAMMENSETZUNG

COMPOSITION DE SOIN CAPILLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.10.2013 PCT/CN2013/085505**
**19.12.2013 EP 13198344**

(43) Date of publication of application:
**24.08.2016 Bulletin 2016/34**

(73) Proprietors:
• **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
• **Unilever PLC**
**London, Greater London EC4Y 0DY (GB)**
Designated Contracting States:
**CY GB IE MT**

(72) Inventors:
• **JAYASWAL, Amit**
**Bihar 811201 (IN)**
• **PI, Yingying**
**Shanghai 200335 (CN)**
• **LI, Zhengrong**
**Shanghai 200335 (CN)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(56) References cited:
WO-A1-03/094874        WO-A1-2004/043414
WO-A1-2013/011122      US-A1- 2004 076 595
US-A1- 2012 309 733

Remarks:
The file contains technical information submitted after the application was filed and not included in this specification

Description

**TECHNICAL FIELD OF THE INVENTION**

This invention is concerned with hair care compositions containing conditioning agent and cationic deposition polymer. In particular the present invention relates to hair care compositions comprising a combination of cationic deposition polymers. Moreover, the present invention also relates to the use of such hair care compositions for providing cleaning and conditioning benefits to the hair.

**BACKGROUND OF THE INVENTION**

Hair care compositions which provide a combination of cleansing and conditioning benefits to the hair are known in the art Such compositions typically comprise one or more anionic cleansing surfactants in combination with one or more conditioning agents. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry.

Cationic polymers are often used to enhance the deposition of the conditioning agent onto the hair. These polymers may be synthetic or natural polymers that have been modified with cationic substituents.

WO 2004/043414 A1 (UNILEVER) discloses hair washing compositions which comprise cleansing surfactant, water, first and second cationic polymers having at pH 7 a charge density from 0.2 to 1 meq/gm and from 1.3 to 3 meq/gm respectively, and droplets of a water-insoluble conditioning oil of diameter 4 micronmetres or less.

WO 2013/011122 A1 (RHODIA OPERATIONS) discloses a non-cellulosic polysaccharide derivative: i) having a mean average molecular weight (Mw) from about 100,000 g/mol, preferably from about 150,000 g/mol and more preferably from about 200,000 g/mol to about 2,000,000 g/mol, preferably to about 1,800,000 g/mol and more preferably to about 1,400,000 g/mol; and ii) containing at least one cationic group, with a cationic degree of substitution $(DS_{cat})_{extraction}$ from about 0.20 to about 0.30.

US 2004/0076595 A1 (THE PROCTER & GAMBLE COMPANY) discloses a hair conditioning composition comprising: (a) a thickening polymer system selected from the group consisting of a cationic thickening polymer, a nonionic thickening polymer, and mixtures thereof; (b) cationic surfactant system selected from the group consisting of one cationic surfactant, a mixture of two or more cationic surfactants, and a mixture of a cationic surfactant and a nonionic surfactant; and (c) an aqueous carrier; wherein the composition is substantially free of a water-insoluble high melting point oily compound and an anionic compound.

A problem associated with the use of cationic deposition polymers is that it is difficult to obtain a desirable hair feel and hair appearance while retaining high conditioning benefits. For example, some cationic polymers are effective at improving wet stage hair sensory attributes during the stages of washing and rinsing but give an undesirable hair feel after drying such as the heavy, greasy, coated feel or significantly reduced hair volume that many consumers experience when high charge density cationic polymers and oily conditioning agents are combined in hair care compositions.

The present inventors have now recognized a need to develop a hair care composition that can provide high conditioning benefits while maintaining a pleasant hair feel after drying. It has been found that this need can be met by using a combination of two cationic polymers of specific molecular weight and cationic charge densities in a hair care composition, which also comprises small droplets of a water-insoluble oily conditioning agent. Moreover, the hair care composition of the present invention demonstrates unaffected deposition efficiency of antidandruff actives onto scalp which indicates it can also be used for antidandruff purposes.

**DEFINITIONS**

Degree of Substitution

"Degree of substitution", as used herein, refers to the average number of moles of cationic groups per mole of sugar unit. The degree of substitution (DS) is measured using $^1$H NMR in a solvent of deuterium oxide ($D_2O$) and deuterium chloride (DCl) mixture. For example, the DS of guar hydroxylpropyltrimonium chloride is measured using $^1$H NMR and the spectrum is recorded at 25°C. The peak corresponding to the nine methyl protons of the quaternary ammonium group on guar units, which appears between 3.1-3.3 ppm, is integrated as A1. The multiplet of peaks corresponding to the anomeric protons on sugar ring and protons on $CH_2$ and CH groups of the cationic substituent, which appear between 3.3-4.5 ppm, are also integrated as A2. Therefore, the DS for the case of the cationizing agent 2,3-epoxypropyltrimethylammonium chloride may be calculated as follows:

$$DS= \frac{(A1/9)}{(A2-A1\times5/9)/6}$$

### Cationic Charge Density

"Cationic charge density", as used herein, refers to the number of cationic charges per weight unit of a given polymer. Cationic charge density can be calculated from the degree of substitution as described in WO 2013/011122, the disclosure of which is hereby incorporated by reference in its entirety but especially page 8 lines 8-17. For example, for cationically-modified guar polymer obtained by reacting with 2,3-epoxypropyltrimethylammonium chloride, the cationic charge density may be calculated from the DS using the following equation:

$$\text{Cationic charge density in milliequivalents per gram (meq/g)}= \frac{DS\times1000}{162+151\times DS}$$

### High Charge Density Cationic Polymer

"High charge density cationic polymer", as used herein, refers to the cationic polymers which have cationic charge densities of greater than 1.2 meq per gram.

### Water-Insoluble

"Water-insoluble", as used herein, refers to the solubility of a material in water at 25°C and atmospheric pressure being 0.1 % by weight or less.

### Molecular Weight

"Molecular weight", as used herein, refers to the weight average molecular mass of a given polymer, preferably measured by SEC-MALS (Size Exclusion Chromatography with detection by Multi-Angle Light-Scattering detection) as described in WO 2013/011122, the disclosure of which is hereby incorporated by reference in its entirety especially page 3 lines 11-18.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

## SUMMARY OF THE INVENTION

In a first aspect, the present invention is concerned with a hair care composition comprising:

    a) cationic deposition polymer; and
    b) discrete dispersed droplets of a water-insoluble conditioning agent;
    wherein the cationic deposition polymer comprises at least 90% by weight cationically-modified guar by weight of the cationic deposition polymer and wherein the cationically-modified guar comprises:

(i) first cationically-modified guar polymer having a charge density of greater than 1.2 meq/g and a molecular weight of at least 1 million g/mol; and

(ii) second cationically-modified guar polymer having a charge density of from 0.9 to 1.2 meq/g and a molecular weight of at least 1 million g/mol, wherein the composition additionally comprises a surface active block polymer selected from poloxamer or poloxamine; wherein the composition comprises particulate anti-dandruff agent; and wherein the cationic charge density is calculated from the degree of substitution using the following equation: cationic charge density in milliequivalents per gram (meq/g)=(DSx1000)/(162+151xDS); and wherein the degree of substitution is measured using $^1$H NMR and the spectrum is recorded at 25°C.

In a second aspect, the present invention is directed to a packaged hair care product comprising the hair care composition of the first aspect of this invention.

In a third aspect, the present invention is directed to a process for manufacturing any embodiment of the hair care composition of the first embodiment comprising the steps of:

i) selecting the first cationically-modified guar polymer having a charge density of greater than 1.2 meq/g and a molecular weight of at least 1 million g/mol;

ii) selecting the second cationically-modified guar polymer having a charge density of from 0.8 to 1.2 meq/g and a molecular weight of at least 1 million g/mol;

iii) combining the first and second cationically-modified guar polymers with discrete droplets of a water-insoluble conditioning agent.

In a fourth aspect, the present invention is concerned with a hair care composition obtainable and/or obtained by a process of the third aspect.

In a fifth aspect, the present invention is directed to a method of using hair care composition of the first aspect of this invention to provide high conditioning benefits while maintaining a pleasant hair feeling after drying.

All other aspects of the present invention will more readily become apparent upon considering the detailed description and examples which follow.

## DETAILED DESCRIPTION

Now it has been found that a hair care composition comprising a combination of two cationic polymers of specific molecular weight and cationic charge densities together with small droplets of a water-insoluble oily conditioning agent can provide high conditioning benefits to the hair while still maintaining a pleasant hair feel after drying. Moreover, the hair care composition of the present invention can also be used for antidandruff purposes.

CATIONIC DEPOSITION POLYMERS

The cationic polymer suitable for use in compositions of the present invention comprises cationically-modified guar polymers such as guar hydroxypropyltrimonium chloride, which is, for example, commercially available from Rhodia in their JAGUAR trademark series.

Generally for cationic polysaccharide polymers, the hydroxyl groups of the non-modified monomeric sugar ring units are the sites for cationic substitution. Degree of substitution (DS) is typically in the range from 0 to 3 due to the fact that the monomeric sugar unit of most polysaccharide has in average three hydroxyl groups available for substitution. In addition to the DS, the cationic charge on polymers can also be quantified as cationic charge density. DS has previously been determined by different methods. For example, the cationic charge density of the polymer has in some cases been calculated based on a percent nitrogen content determined via the Kjeldahl method as described in US Pharmacopoeia under chemical tests for nitrogen determination and is expressed in milliequivalents (meq) per gram. The cationic charge density of the polymer in the present invention is, however, calculated from the degree of substitution, which is measured by $^1$H NMR in a solvent of deuterium oxide (D$_2$O) and deuterium chloride (DCl) mixture.

In many cases the DS obtained from $^1$H NMR measurement may not be suitable to be compared with that obtained from Kjeldahl method, due to the fact that the two methods are influenced by different factors.

The cationic deposition polymer according to the invention comprises a combination of two cationic polymers. The two cationic polymers are cationically-modified guar polymers in an amount of at least 90% by weight of the cationic deposition polymer, more preferably at least 95%, even more preferably at least 98% and most preferably the cationic deposition polymer consists (or at least consists essentially) of cationically-modified guar polymers.

The first cationically-modified guar polymer suitably has a charge density of at least 1.2 meq per gram, preferably from 1.3 to 1.8 meq per gram. Suitably the first cationically-modified guar polymer has a mean molecular weight of at least 1 million gram per mole, preferably from 1.1 to 3 million gram per mole. An example of such a polymer is described as polymer 2 in Table 1 of WO 2013/011122. The second cationically-modified guar polymer suitably has a charge density from 0.8 to 1.2 meq per gram, preferably from 0.9 to 1.1 meq per gram. Suitably the second cationically-modified guar polymer has a mean molecular weight of at least 1 million gram per mole, preferably from 1.5 to 3 million gram per mole. A specific example of such a polymer is JAGUAR C17.

Typically, the hair care composition of the present invention comprises the first cationically-modified guar polymer in an amount from 0.001 to 1% by weight of the hair care composition, preferably from 0.005 to 0.8%, more preferably from 0.01 to 0.5%, most preferably from 0.03 to 0.3%, based on the total weight of the hair care composition and including all ranges subsumed therein.

Typically, the hair care composition of the present invention comprises the second cationically-modified guar polymer in an amount from 0.001 to 1% by weight of the hair care composition, preferably from 0.005 to 0.8%, more preferably from 0.01 to 0.5%, most preferably from 0.03 to 0.3%, based on the total weight of the hair care composition and including all ranges subsumed therein.

Generally, the hair care composition of the present invention comprises the first and second cationically-modified guar polymer in a total amount from 0.002 to 2% by weight of the hair care composition, preferably from 0.01 to 1.6%, more preferably from 0.02 to 1%, most preferably from 0.06 to 0.6%, based on the total weight of the hair care composition and including all ranges subsumed therein.

The relative weight ratio of the first cationically-modified guar polymer to the second cationically-modified guar polymer in the hair care composition of the this invention may range from 1:15 to 15:1, preferably from 1:10 to 10:1, most preferably from 1:5 to 5:1.

In addition to the first and second cationic guars, the deposition polymer may comprise a minor amount of further cationic polymers. The further cationic polymer may be a homopolymer or be formed from two or more types of monomers. The molecular weight of the polymer will generally be between 5,000 and 10,000,000 gram per mole, typically at least 10,000 and preferably from 100,000 to 2,000,000. The polymers will have cationic nitrogen containing groups such as quaternary ammonium or protonated amino groups, or a mixture thereof.

The cationic nitrogen containing group will generally be present as a substituent on a fraction of the total monomer units of the cationic polymer. The ratio of the cationic to non-cationic monomer units is selected to give polymers having a cationic charge density in the required range.

Suitable further cationic polymers include, for example, copolymers of vinyl monomers having cationic amine or quaternary ammonium functionalities with water soluble spacer monomers such as (meth)acrylamide, alkyl and dialkyl (meth) acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol. Preferably, the further cationic polymer is cationic polysaccharide polymers, such as cationic cellulose derivatives, cationic starch derivatives and mixtures thereof.

CONDITIONING AGENT

The hair care composition of this invention comprises a water-insoluble conditioning agent to enhance conditioning performance. Preferably, the conditioning agent is non-volatile, meaning that it has a vapour pressure of less than 1000 Pa at 25°C.

Preferably, the hair care composition comprises discrete dispersed droplets of a water-insoluble conditioning agent, which has a mean droplet diameter $(D_{3,2})$ of less than 15 microns, preferably less than 10 microns, more preferably less than 5 microns, most preferably less than 3 microns. The mean droplet diameter $(D_{3,2})$ of a water-insoluble conditioning agent may be measured by means of a laser light scattering technique, for example using a 2600D Particle Sizer from Malvern Instruments.

The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of cross-linking, as are described for example in WO 96/31188. Preferably, the silicone oil comprises dimethicone, dimethiconol or a mixture thereof.

The viscosity of the emulsified silicone itself (not the emulsion or the final hair care composition) is typically at least 10,000 cSt (centi-Stokes=$mm^2 \cdot S^{-1}$) at 25°C, preferably at least 60,000 cSt, most preferably at least 500,000 cSt, ideally at least 1,000,000 cSt. Preferably the viscosity does not exceed $10^9$ cSt for ease of formulation. Suitable methods for measuring the kinematic viscosity of silicone oils are known to those skilled in the art, e.g. capillary viscometers. For high viscosity silicones, a constant stress rheometer can be used to measure viscosity.

Suitable emulsified silicones for use in the hair care compositions of this invention are available as pre-formed silicone emulsions from suppliers of silicones such as Dow Corning and GE silicones. The use of such pre-formed silicone emulsion is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer.

Examples of suitable pre-formed silicone emulsions include DC1785, DC1788, DC7128, all available from Dow Corning. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

Silicone emulsion droplets are blended with certain types of surface active block polymers of a high molecular weight to form silicone emulsions, as described for example in WO03/094874. One preferred form of the surface active block polymer having polyoxypropylene and polyoxyethylene groups as the hydrophobic and hydrophilic part respectively has formula I and has the CTFA designation poloxamer, known commercially under the trade name "Pluronic" from BASF.

$$\text{I)} \qquad HO(CH_2CH_2O)_x(CH(CH_3)CH_2O)_y(CH_2CH_2O)_x H$$

Suitably, the mean value of x in formula I is 4 or more, preferably 8 or more, more preferably 25 or more, yet more preferably 50 or more and most preferably 80 or more. The mean value of x is typically no greater than 200. Suitably, the mean value of y is 25 or more, preferably 35 or more, more preferably 45 or more and most preferably 60 or more. The mean value of y is typically no greater than 100.

Another preferred form of the surface active block polymer is according to formula II and has the CTFA designation Poloxamine. Those are commercially available under the trade name "Tetronic" from BASF.

$$\text{II)} \qquad (HO(CH_2CH_2O)_a(CH(CH_3)CH_2O)_b)_2\text{-N-}CH_2\text{-}CH_2\text{-N-}((OCH_2CH(CH_3))_b(OCH_2CH_2)_a OH)_2$$

Suitably, the mean value of a is 2 or more, preferably 4 or more, more preferably 8 or more, even more preferably 25 or more and most preferably 40 or more. The mean value of a is typically no greater than 200. The mean value of b is suitably 6 or more, preferably 9 or more, more preferably 11 or more and most preferably 15 or more. The mean value of b is typically no greater than 50.

The surface active block polymer is poloxamer and/or poloxamine, more preferably, the surface active block polymer is poloxamer.

Preferably, the surface active block polymer is blended with dimethicone. The weight ratio of dimethicone to surface active block polymer in the blend is preferably in the range from 2:1 to 200:1, more preferably from 5:1 to 50:1, even more preferably from 10:1 to 40:1, most preferably from 15:1 to 30:1.

The water-insoluble conditioning agent is generally present in hair care composition of this invention in an amount from 0.05 to 15%, preferably from 0.1 to 10%, more preferably from 0.5 to 8%, most preferably from 1 to 5%, based on the total weight of the hair care composition and including all ranges subsumed therein.

CLEANSING SURFACTANT

In a preferred embodiment, the hair care composition according to the invention comprises one or more cleansing surfactants. Preferably, the cleansing surfactants are anionic surfactants.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in hair care compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective

formulae $R_2OSO_3M$ and $R_1O(C_2H_4O)_xSO_3M$, wherein $R_2$ is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably $R_2$ has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Generally, the total amount of cleansing surfactant in hair care composition of the present invention ranges from 0.5 to 45%, more preferably from 1.5 to 30%, most preferably from 5 to 20%, based on the total weight of the hair care composition and including all ranges subsumed therein.

In an especially preferred embodiment, the hair care composition may further comprise co-surfactants such as amphoteric and zwitterionic surfactants to provide mildness to the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in compositions of the invention include lauryl amine oxide, cocodimethyl sulphopropyl betaine, lauryl betaine, cocamidopropyl betaine and sodium cocoamphoacetate. Preferably, the co-surfactant is cocamidopropyl betaine (CAPB).

Typically, co-surfactant may be present in hair care compositions of the invention in an amount from 0.5 to 8% by weight of the hair care composition, preferably from 1 to 4%, based on the total weight of the hair care composition and including all ranges subsumed therein.

ANTIDANDRUFF ACTIVES

The hair care composition comprises antidandruff agents. Antidandruff agents are compounds that are active against dandruff and are typically antimicrobial agents and preferably antifungal agents.

Suitable antidandruff agents include compounds selected from azole based antifungal agents, octopirox, metal pyrithione salts, selenium sulfide and mixtures thereof. The preferred azole based antifungal agents are ketoconazole and climbazole. Preferred metal pyrithione salts are zinc, copper, silver and zirconium pyrithione. Most preferred are particulate antidandruff actives zinc pyrithione and selenium sulfide.

Typically, the hair care composition of the invention comprises antidandruff agent in an amount ranging from 0.01 to 10%, preferably from 0.05 to 5%, most preferably from 0.1 to 2%, based on the total weight of the hair care composition and including all ranges subsumed therein.

OTHER INGREDIENTS

The hair care composition of the present invention may contain other ingredients which are common in the art to enhance physical properties and performances. Suitable ingredients include but are not limited to fragrance, suspending agents, dyes and pigments, pH adjusting agents, pearlescers or opacifiers, viscosity modifiers, thickeners, preservatives, antimicrobials and natural hair nutrients such as botanicals, fruit extracts, sugar derivatives and amino acids.

The compositions of the invention are primarily intended for topical application to scalp and/or at least a portion of the hair of an individual, either in rinse-off or leave-on compositions, for the treatment of dry and/or wet, damaged and/or unmanageable hair.

The following examples are provided to facilitate an understanding of the present invention. The examples are not provided to limit the scope of the claims.

EXAMPLES

Compositions were prepared according to the formulations detailed in Table 1. All ingredients are expressed by amount of active in percent by weight of the composition.

Jaguar C17 is a commercial cationic guar from Rhodia which has a molecular weight of 2,000,000 g/mol and a cationic charge density of 1.0 meq/g.

HD1 is a high charge density (HD) cationic guar made by Rhodia which has a molecular weight of 1,200,000 g/mol and a cationic charge density of 1.4 meq/g, similar to polymer 2 described in Table 1 of WO 2013/011122.

HD2 is a HD guar made by Rhodia which has a molecular weight of 370,000 g/mol and a cationic charge density of 1.4 meq/g, similar to polymer 4 described in Table 1 of WO 2013/011122.

DC1788 is a commercial dimethiconol from Dow corning which has a particle size of 0.2 $\mu$m.

DC7128 is a commercial dimethicone pre-blended with poloxamer from Dow corning which has a particle size of 10μm.

DM is a dimethicone pre-blended with poloxamer made by Dow corning which is similar to DC7128 but with a particle size of 2μm.

TABLE 1

| Ingredient | Samples | | | | | | | | | |
|---|---|---|---|---|---|---|---|---|---|---|
| | 1 | 2 | 3 | 4 | 5 | 6 | 7 | 8 | 9 | 10 |
| Sodium Laureth Sulphate | 14 | 14 | 14 | 14 | 14 | 14 | 14 | 14 | 14 | 14 |
| Cocoamidopropyl Betaine | 1.6 | 1.6 | 1.6 | 1.6 | 1.6 | 1.6 | 1.6 | 1.6 | 1.6 | 1.6 |
| Guar Hydroxypropyltrimonium Chloride (Jaguar C17) | 0.2 | 0.05 | 0.05 | 0.15 | -- | 0.2 | -- | 0.2 | 0.15 | 0.05 |
| Guar Hydroxypropyltrimonium Chloride (HD1) | -- | 0.15 | 0.15 | 0.05 | 0.2 | -- | -- | -- | 0.05 | 0.15 |
| Guar Hydroxypropyltrimonium Chloride (HD2) | -- | -- | -- | -- | -- | -- | 0.2 | -- | -- | -- |
| Zinc Pyrithione | 1.0 | 1.0 | 1.0 | 1.0 | 1.0 | 1.0 | 1.0 | 1.0 | 1.0 | 1.0 |
| Climbazole | -- | -- | -- | -- | -- | -- | -- | 0.5 | 0.5 | 0.5 |
| Zinc Sulphate | 0.1 | 0.1 | 0.1 | 0.1 | 0.1 | 0.1 | 0.1 | 0.1 | 0.1 | 0.1 |
| Dimethiconol (DC1788) | 1.8 | 1.8 | 1.8 | 1.8 | 1.8 | 1.2 | 1.8 | 1.2 | 1.2 | 1.2 |
| Dimethicone (DC7128) | 1.2 | 1.2 | -- | 1.2 | 1.2 | 0.8 | 1.2 | 0.8 | 0.8 | -- |
| Dimethicone (DM) | -- | -- | 1.2 | -- | -- | -- | -- | -- | -- | 0.8 |
| Perfume | 0.7 | 0.7 | 0.7 | 0.7 | 0.7 | 0.7 | 0.7 | 0.7 | 0.7 | 0.7 |
| Sodium Chloride | 0.5 | 0.5 | 0.5 | 0.5 | 0.5 | 0.5 | 0.5 | 0.5 | 0.5 | 0.5 |
| Acrylic Acid Polymer (Carbomer) | 0.6 | 0.6 | 0.6 | 0.6 | 0.6 | 0.6 | 0.6 | 0.6 | 0.6 | 0.6 |
| Preservative | 0.2 | 0.2 | 0.2 | 0.2 | 0.2 | 0.2 | 0.2 | 0.2 | 0.2 | 0.2 |
| Water | Balance | Balance | Balance | Balance | Balance | Balance | Balance | Balance | Balance | Balance |

## Example 1

This example demonstrates the silicone deposition, silicone build-up on hair samples, hair dry friction and hair volume after treatment with hair care compositions.

Methods

*Silicone Deposition and Silicone Build-up*

Hair switches were soaked in sodium lauryl ether sulphate (SLES) for a few minutes before being thoroughly rinsed under tap water. The hair switches were combed until the fibers were aligned and then were allowed to dry overnight.

The hair switches were then held together and pre-wetted with water, followed by removing the excess water. Shampoo was applied to the five switches, and the switches were lathered to incorporate shampoo evenly. The switches were rinsed under tap water before removing the excess water. For silicone deposition measurement, the shampoo washing and water rinsing steps were repeated one more time. But for silicone build-up measurement, the same steps were repeated eight times. The ratio of silicone deposition after eight washes to silicone deposition after one wash was recorded as silicone build-up in Table 2. The hair switches were combed through until the fibers were aligned, and dried overnight for the measurement.

*Hair Dry Friction*

The hair switch was clamped onto the bed of the texture analyser (TA) to ensure a flat surface with little or no stray hair fibers. The probe cylinder covered with rubber sleeve was mounted onto the probe arm and lowered onto the switch. 500 gram weight was placed on the platform of the probe arm and the force spent to move the probe arm along with the hair switch was recorded.

*Hair Volume*

The volume of hair switches after washing was determined quantitatively using an instrumental method. This involved taking images of each switch held in a vertical position under controlled lighting conditions and subjecting the resulting images to image analysis.

TABLE 2

| Samples | Dry friction/g*mm | Silicone deposition/ppm | Silicone build-up/ppm | Volume/mm$^2$ |
|---|---|---|---|---|
| **1** | 22933 | 2148 | 2.04 | 7871 |
| **2** | 19743 | 3542 | 1.91 | 7656 |
| **4** | 21178 | NM[a] | 1.8 | 8318 |
| **5** | 20513 | 2891 | NM[a] | NM[a] |
| a. NM means that the data is not measured. | | | | |

Results

Table 2 shows that Sample 2 with a high level of HD guar had comparable amount of silicone build-up and comparable hair volume to Sample 1 but smaller hair dry friction, which demonstrated that Sample 2 had parity clean performance to Sample 1 but provided better conditioning benefits. Sample 4 with a lower level of HD guar had comparable hair dry friction to Sample 1 but smaller amount of silicone build-up and larger hair volume, which demonstrated that Sample 4 had parity hair conditioning performance to Sample 1 but better cleaning benefits. Sample 5 where only HD guar was present, demonstrated better conditioning performance than Sample 1 but not to Sample 2 in terms of hair dry friction.

## Example 2

This example demonstrates the effect of different conditioning agents in hair care compositions.

Two formulations with same ratio of cationic guars but different conditioning agent were scored by 36 consumers.

The results of the evaluation are shown in Table 3 and are significant to the 99% confidence level.

TABLE 3

| Attribute | | Samples | |
|---|---|---|---|
| | | **2** | **3** |
| **Wet stage** | Easier wet combing-total effect | 5 | 30 |
| | Easier wet combing-single stroke | 6 | 30 |
| | More slippery feel wet | 6 | 30 |
| | More coating wet | 6 | 29 |
| **Dry stage** | Easier dry combing-total effect | 4 | 31 |
| | Easier dry combing-single stroke | 5 | 31 |
| | Volume | 32 | 4 |
| | Palm smooth | 31 | 5 |
| | More visual expansion from scalp | 31 | 5 |
| | Less stick out | 5 | 31 |
| | More shiny | 31 | 5 |
| | More slippery feel | 5 | 31 |
| | Hair surface smoother | 5 | 31 |
| | More elastic when compressed | 31 | 5 |
| | Higher hair dryness | 31 | 5 |
| | More coating | 5 | 31 |
| | Finger through | 5 | 31 |
| | More weighty hair | 5 | 31 |
| | More sticky | 5 | 31 |
| | Static (easier) | 31 | 5 |

The numbers listed in the table are number of consumers who agreed with the statement. The data clearly shows that Sample 3 which had a dimethicone with a particle size of $2 \mu$m gave superior wet and dry conditioning performance to Sample 2, which had a silicone of a larger size. This trend was consistent through all the different hair types. The consumers expressed a clear preference for Sample 3 on its conditioning benefits.
Similar consumer study was carried out for Sample 1 and Sample 3. The results are summarized in Table 4 and are significant to the 99% confidence level.

TABLE 4

| Attribute | | Samples | |
|---|---|---|---|
| | | **1** | **3** |
| **Wet stage** | Easier wet combing-total effect | 6 | 27 |
| | Easier wet combing-single stroke | 6 | 29 |
| | More slippery feel wet | 6 | 29 |
| | More coating wet | 6 | 29 |

(continued)

| Attribute | | Samples | |
|---|---|---|---|
| | | 1 | 3 |
| Dry stage | Easier dry combing-total effect | 7 | 28 |
| | Easier dry combing-single stroke | 7 | 29 |
| | Volume | 29 | 7 |
| | Palm smooth | 29 | 7 |
| | More visual expansion from scalp | 29 | 6 |
| | Less stick out | 7 | 29 |
| | More shiny | 7 | 7 |
| | More slippery feel | 7 | 29 |
| | Hair surface smoother | 7 | 29 |
| | More elastic when compressed | 29 | 7 |
| | Higher hair dryness | 29 | 7 |
| | More coating | 7 | 29 |
| | Finger through | 7 | 29 |
| | More weighty hair | 7 | 29 |
| | More sticky | 7 | 29 |
| | Static (easier) | 29 | 7 |

The data demonstrated that Sample 3 also provided significantly better wet and dry conditioning benefits when compared to Sample 1.

### Example 3

This example demonstrates the cleaning benefits of compositions comprising HD guar and silicone.

Sample 4 comprising HD guar and a larger amount of silicone was compared against a benchmark formulation Sample 6 comprising only one cationic guar (Jaguar C17) and a smaller amount of silicone.

Each formulation was scored by 100 users for a set of performance attributes and the attributes reported in Table 5 show significant differences to the 95% confidence level. The numbers recorded were the percentage of people who give a positive answer towards particular attributes on specific formulation.

TABLE 5

| Attributes | Samples | |
|---|---|---|
| | 4 | 6 |
| Cleans my hair well | 94% | 88% |
| Cleans my hair and my scalp thoroughly | 93% | 84% |

It is known that silicones are conditioning agents, which deposit onto hair. But the build-up of silicone deposition can generate unsatisfying cleaning performance. Surprisingly, Sample 4 with a larger amount of silicone outperformed Sample 6 at cleaning performance. Sample 4 was preferred over the benchmark on cleaning hair and scalp.

### Example 4

This example demonstrates the deposition of antidandruff agent zinc pyrithione (ZnPTO) onto the scalp.

In-vitro skin was treated with shampoo and water, washed by manual rubbing with a plastic stick then the liquid was removed. The washing process was repeated again using water followed by removing the liquid. The in-vitro skin was then allowed to dry overnight. The ZnPTO deposition on in-vitro skin was measured by X-ray fluorescence (XRF). The results are reported in Table 6 and Table 7.

TABLE 6

| Samples | 8 | 9 |
|---|---|---|
| ZnPTO Deposition [a] ($\mu$g/cm$^2$) | 7.449 | 7.598 |
| a. Zinc pyrithione | | |

TABLE 7

| Samples | 1 | 5 | 7 |
|---|---|---|---|
| ZnPTO Deposition($\mu$g/cm$^2$) | 5.890 | 5.727 | 3.737 |

The data in Table 6 and Table 7 demonstrated that the incorporation of HD guar into a hair care composition did not affect the deposition of ZnPTO. The ZnPTO deposition remained consistent even for Sample 5 where only HD guar was present. However, Sample 7 comprising HD2 which had the same charge density as HD1 but a much smaller molecular weight, showed inferior deposition of ZnPTO.

**Claims**

1.    A hair care composition comprising:

    a) cationic deposition polymer; and
    b) discrete dispersed droplets of a water-insoluble conditioning agent; wherein the cationic deposition polymer comprises at least 90% by weight cationically-modified guar by weight of the cationic deposition polymer and wherein the cationically-modified guar comprises:

        (i) first cationically-modified guar polymer having a charge density of greater than 1.2 meq/g and a molecular weight of at least 1 million g/mol; and
        (ii) second cationically-modified guar polymer having a charge density of from 0.9 to 1.2 meq/g and a molecular weight of at least 1 million g/mol.

    Wherein the composition additionally comprises a surface active block polymer selected from poloxamer or poloxamine;
    wherein the composition comprises particulate anti-dandruff agent; and wherein the cationic charge density is calculated from the degree of substitution using the following equation:

$$\text{cationic charge density in milliequivalents per gram (meq/g)} = \frac{DS \times 1000}{162 + 151 \times DS};$$

    and wherein the degree of substitution is measured using $^1$H NMR and the spectrum is recorded at 25°C.

2.    The hair care composition as claimed in claim 1 wherein the charge density of the second cationically-modified guar polymer is from 0.9 to 1.1 meq/g.

3.    The hair care composition as claimed in claim 1 or claim 2 where in the molecular weight of the second cationically-modified guar polymer is from 1.5 to 3 million g/mol.

4.    The hair care composition as claimed in any one of the preceding claims wherein the charge density of the first cationicaily-modified guar polymer is from 1.3 to 1.8 meq/g.

5.    The hair care composition as claimed in any one of the preceding claims wherein the molecular weight of the first cationically-modified guar polymer is from 1.1 to 3 million g/mol.

6. The hair care composition as claimed in any one of the preceding claims wherein the composition comprises the first cationically-modified guar polymer and the second cationically modified guar polymer in a weight ratio (first:second) of from 1:10 to 10:1.

7. The hair care composition as claimed in any one of the preceding claims wherein the composition comprises the first and second cationically-modified guar polymers in a total amount from 0.02 to 1 % by weight of the composition.

8. The hair care composition as claimed in any one of the preceding claims wherein the droplets of conditioning agent have a mean diameter (D3,2) of less than 5 microns, and wherein the mean droplet diameter (D3,2) of a water-insoluble conditioning agent is measured by means of a laser light scattering technique.

9. The hair care composition as claimed in any one of the preceding claims wherein the conditioning agent comprises silicone oil, preferably dimethicone, dimethiconol or a mixture thereof.

10. The hair care composition as claimed in any one of the preceding claims wherein the surface active block polymer is poloxamer.

11. The hair care composition as claimed in any one of the preceding claims wherein the surface active block polymer is blended with silicone oil, preferably dimethicone..

12. The hair care composition as claimed in any one of the preceding claims wherein the composition comprises the water-insoluble conditioning agent in an amount of from 0.1 to 10% by weight of the composition.

13. The hair care composition as claimed in any one of the preceding claims wherein the particulate anti-dandruff agent is particulate metal pyrithione salt.

14. The hair care composition as claimed in any of the preceding claims, wherein the composition comprises particulate anti-dandruff agent in an amount of from 0.05 to 5% by weight of the composition.

15. The hair care composition as claimed in any one of the preceding claims wherein the composition comprises anionic cleansing surfactant, preferably in an amount ranging from 5 to 20% by weight of the hair care composition.

16. A process for manufacturing the hair care composition as claimed in any one of the claims 1 to 15 wherein the process comprises the steps of:

i) selecting the first cationically-modified guar polymer having a charge density of greater than 1.2 meq/g and a molecular weight of at least 1 million g/mol;
ii) selecting the second cationically-modified guar polymer having a charge density of from 0.8 to 1.2 meq/g and a molecular weight of at least 1 million g/mol;
iii) combining the first and second cationically-modified guar polymers with discrete droplets of a water-insoluble conditioning agent.

**Patentansprüche**

1. Haarpflegezusammensetzung, umfassend:

a) kationisches Abscheidungspolymer; und
b) diskrete dispergierte Tröpfchen eines wasserunlöslichen Konditionierungsmittels;

wobei das kationische Abscheidungspolymer mindestens 90 Gew.-% kationisch modifiziertes Guar, bezogen auf das Gewicht des kationischen Abscheidungspolymers, umfasst und wobei das kationisch modifizierte Guar Folgendes umfasst:

(i) erstes kationisch modifiziertes Guarpolymer mit einer Ladungsdichte von mehr als 1,2 mÄq/g und einem Molekulargewicht von mindestens 1 Million g/mol; und

(ii) zweites kationisch modifiziertes Guarpolymer mit einer Ladungsdichte von 0,9 bis 1,2 mÄq/g und einem Molekulargewicht von mindestens 1 Million g/mol,

wobei die Zusammensetzung zusätzlich ein oberflächenaktives Blockpolymer ausgewählt aus Poloxamer oder Poloxamin umfasst,
wobei die Zusammensetzung teilchenförmiges Antischuppenmittel umfasst, und wobei die kationische Ladungsdichte aus dem Substitutionsgrad unter Verwendung der folgenden Gleichung berechnet wird:

$$\text{kationische Ladungsdichte in Milliäquivalenten pro Gramm (mÄq/g)} = \frac{DS \times 1000}{162 + 151 \times DS}$$

und wobei der Substitutionsgrad unter Verwendung von $^1$H-NMR gemessen wird und das Spektrum bei 25 °C aufgezeichnet wird.

2. Haarpflegezusammensetzung wie in Anspruch 1 beansprucht, wobei die Ladungsdichte des zweiten kationisch modifizierten Guarpolymers 0,9 bis 1,1 mÄq/g beträgt.

3. Haarpflegezusammensetzung wie in Anspruch 1 oder Anspruch 2 beansprucht, wobei das Molekulargewicht des zweiten kationisch modifizierten Guarpolymers 1,5 bis 3 Millionen g/mol beträgt.

4. Haarpflegezusammensetzung wie in irgendeinem der vorhergehenden Ansprüche beansprucht, wobei die Ladungsdichte des ersten kationisch modifizierten Guarpolymers 1,3 bis 1,8 mÄq/g beträgt.

5. Haarpflegezusammensetzung wie in irgendeinem der vorhergehenden Ansprüche beansprucht, wobei das Molekulargewicht des ersten kationisch modifizierten Guarpolymers 1,1 bis 3 Millionen g/mol beträgt.

6. Haarpflegezusammensetzung wie in irgendeinem der vorhergehenden Ansprüche beansprucht, wobei die Zusammensetzung das erste kationisch modifizierte Guarpolymer und das zweite kationisch modifizierte Guarpolymer in einem Gewichtsverhältnis (erste : zweite) von 1:10 bis 10:1 umfasst.

7. Haarpflegezusammensetzung wie in irgendeinem der vorhergehenden Ansprüche beansprucht, wobei die Zusammensetzung das erste und zweite kationisch modifizierte Guarpolymer in einer Gesamtmenge von 0,02 bis 1 Gew.-% der Zusammensetzung umfasst.

8. Haarpflegezusammensetzung wie in irgendeinem der vorhergehenden Ansprüche beansprucht, wobei die Tröpfchen des Konditionierungsmittels einen mittleren Durchmesser (D3,2) von weniger als 5 Mikron aufweisen, wobei der mittlere Tröpfchendurchmesser (D3,2) eines wasserunlöslichen Konditionierungsmittels mittels einer Laserlichtstreuungstechnik gemessen wird.

9. Haarpflegezusammensetzung wie in irgendeinem der vorhergehenden Ansprüche beansprucht, wobei das Konditionierungsmittel Siliconöl, bevorzugt Dimethicon, Dimethiconol oder eine Mischung davon, umfasst.

10. Haarpflegezusammensetzung wie in irgendeinem der vorhergehenden Ansprüche beansprucht, wobei das oberflächenaktive Blockpolymer Poloxamer ist.

11. Haarpflegezusammensetzung wie in irgendeinem der vorhergehenden Ansprüche beansprucht, wobei das oberflächenaktive Blockpolymer mit Siliconöl, bevorzugt Dimethicon, gemischt ist.

12. Haarpflegezusammensetzung wie in irgendeinem der vorhergehenden Ansprüche beansprucht, wobei die Zusammensetzung das wasserunlösliche Konditionierungsmittel in einer Menge von 0,1 bis 10 Gew.-% der Zusammensetzung umfasst.

13. Haarpflegezusammensetzung wie in irgendeinem der vorhergehenden Ansprüche beansprucht, wobei das teilchenförmige Antischuppenmittel teilchenförmiges Metallpyrithionsalz ist.

14. Haarpflegezusammensetzung wie in irgendeinem der vorhergehenden Ansprüche beansprucht, wobei die Zusammensetzung teilchenförmiges Antischuppenmittel in einer Menge von 0,05 bis 5 Gew.-% der Zusammensetzung umfasst.

**15.** Haarpflegezusammensetzung wie in irgendeinem der vorhergehenden Ansprüche beansprucht, wobei die Zusammensetzung anionisches Reinigungstensid umfasst, bevorzugt in einer Menge im Bereich von 5 bis 20 Gew.-% der Haarpflegezusammensetzung.

**16.** Verfahren zur Herstellung der Haarpflegezusammensetzung wie in irgendeinem der Ansprüche 1 bis 15 beansprucht, wobei das Verfahren die folgenden Schritte umfasst:

i) Auswählen des ersten kationisch modifizierten Guarpolymers mit einer Ladungsdichte von mehr als 1,2 mÄq/g und einem Molekulargewicht von mindestens 1 Million g/mol;
ii) Auswählen des zweiten kationisch modifizierten Guarpolymers mit einer Ladungsdichte von 0,8 bis 1,2 mÄq/g und einem Molekulargewicht von mindestens 1 Million g/mol;
iii) Kombinieren des ersten und zweiten kationisch modifizierten Guarpolymers mit diskreten Tröpfchen eines wasserunlöslichen Konditionierungsmittels.

**Revendications**

**1.** Composition pour le soin des cheveux comprenant :

a) un polymère de dépôt cationique ; et
b) des gouttelettes dispersées discrètes d'un agent conditionnant insoluble dans l'eau ;

dans laquelle le polymère de dépôt cationique comprend au moins 90 % en masse de guar cationiquement-modifié en masse du polymère de dépôt cationique et dans laquelle le guar cationiquement-modifié comprend :

(i) un premier polymère de guar cationiquement-modifié présentant une densité de charge supérieure à 1,2 meq/g et une masse moléculaire d'au moins 1 million g/mol ; et
(ii) un second polymère de guar cationiquement-modifié présentant une densité de charge de 0,9 à 1,2 meq/g et une masse moléculaire d'au moins 1 million g/mol

dans laquelle la composition comprend de plus un polymère séquencé actif en surface choisi parmi un poloxamère ou une poloxamine ;
dans laquelle la composition comprend un agent antipelliculaire particulaire ; et
dans laquelle la densité de charge cationique est calculée à partir du degré de substitution en utilisant l'équation suivante :

densité de charge cationique en milliéquivalents par gramme

$$(meq/g) = \frac{DS x 1000}{162 + 151 x DS} \ ;$$

et
dans laquelle le degré de substitution est mesuré en utilisant une RMN $^1$H et le spectre est enregistré à 25°C.

**2.** Composition pour le soin des cheveux selon la revendication 1, dans laquelle la densité de charge du second polymère de guar cationiquement-modifié est de 0,9 à 1,1 meq/g.

**3.** Composition pour le soin des cheveux selon la revendication 1 ou la revendication 2, dans laquelle la masse moléculaire du second polymère de guar cationiquement-modifié est de 1,5 à 3 millions g/mol.

**4.** Composition pour le soin des cheveux selon l'une quelconque des revendications précédentes, dans laquelle la densité de charge du premier polymère de guar cationiquement-modifié est de 1,3 à 1,8 meq/g.

**5.** Composition pour le soin des cheveux selon l'une quelconque des revendications précédentes, dans laquelle la masse moléculaire du premier polymère de guar cationiquement-modifié est de 1,1 à 3 millions g/mol.

**6.** Composition pour le soin des cheveux selon l'une quelconque des revendications précédentes, dans laquelle la composition comprend le premier polymère de guar cationiquement-modifié et le second polymère de guar cationiquement-modifié dans un rapport massique (premier : second) de 1:10 à 10:1.

**7.** Composition pour le soin des cheveux selon l'une quelconque des revendications précédentes, dans laquelle la composition comprend les premier et second polymères de guar cationiquement-modifié dans une quantité totale de 0,02 à 1 % en masse de la composition.

**8.** Composition pour le soin des cheveux selon l'une quelconque des revendications précédentes, dans laquelle les gouttelettes d'agent conditionnant présentent un diamètre moyen (D3,2) inférieur à 5 microns, et dans laquelle le diamètre moyen de gouttelettes (D3,2) d'un agent conditionnant insoluble dans l'eau est mesuré au moyen d'une technique de dispersion de lumière laser.

**9.** Composition pour le soin des cheveux selon l'une quelconque des revendications précédentes, dans laquelle l'agent conditionnant comprend une huile de silicone, de préférence de la diméthicone, du diméthiconol ou un mélange de ceux-ci.

**10.** Composition pour le soin des cheveux selon l'une quelconque des revendications précédentes, dans laquelle le polymère séquencé actif en surface est un poloxamère.

**11.** Composition pour le soin des cheveux selon l'une quelconque des revendications précédentes, dans laquelle le polymère séquencé actif en surface est combiné avec une huile de silicone, de préférence de la diméthicone.

**12.** Composition pour le soin des cheveux selon l'une quelconque des revendications précédentes, dans laquelle la composition comprend l'agent conditionnant insoluble dans l'eau dans une quantité de 0,1 à 10 % en masse de la composition.

**13.** Composition pour le soin des cheveux selon l'une quelconque des revendications précédentes, dans laquelle l'agent antipelliculaire particulaire est un sel de pyrithione de métal particulaire.

**14.** Composition pour le soin des cheveux selon l'une quelconque des revendications précédentes, dans laquelle la composition comprend un agent antipelliculaire particulaire dans une quantité de 0,05 à 5 % en masse de la composition.

**15.** Composition pour le soin des cheveux selon l'une quelconque des revendications précédentes, dans laquelle la composition comprend un tensioactif nettoyant anionique, de préférence dans une quantité de 5 à 20 % en masse de la composition pour le soin des cheveux.

**16.** Procédé de fabrication de la composition pour le soin des cheveux selon l'une quelconque des revendications 1 à 15, dans lequel le procédé comprend les étapes de :

i) choix du premier polymère de guar cationiquement-modifié présentant une densité de charge supérieure à 1,2 meq/g et une masse moléculaire d'au moins 1 million g/mol ;
ii) choix du second polymère de guar cationiquement-modifié présentant une densité de charge de 0,8 à 1,2 meq/g et une masse moléculaire d'au moins 1 million g/mol ;
iii) combinaison des premier et second polymères de guar cationiquement-modifié avec des gouttelettes discrètes d'un agent conditionnant insoluble dans l'eau.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004043414 A1
- WO 2013011122 A1
- US 20040076595 A1

- WO 2013011122 A
- WO 9631188 A
- WO 03094874 A